(19) Europäisches Patentamt
European Patent Office
Office européen des brevets

(11) Veröffentlichungsnummer: **0 008 699**
**B1**

(12) # EUROPÄISCHE PATENTSCHRIFT

(21) Anmeldenummer: **79102897.0**

(22) Anmeldetag: **10.08.79**

(51) Int. Cl.³: **C 07 C 65/32,**
C 07 C 51/235

(54) **Verfahren zur Herstellung von Arylglyoxylsäuren**

(30) Priorität: **19.08.78 DE 2836327**

(43) Veröffentlichungstag der Anmeldung:
**19.03.80 Patentblatt 80/06**

(45) Bekanntmachung des Hinweises auf die Patenterteilung:
**07.01.81 Patentblatt 81/01**

(84) Benannte Vertragsstaaten:
**BE CH DE FR GB IT NL**

(56) Entgegenhaltungen:
**DE - A - 1 618 589**
**DE - B - 2 322 105**
**US - A - 3 649 565**

**Chemical Abstracts Band 89, Nr. 21, 20. November 1978 Columbus, Ohio, USA T. MATSUDA et al. "Aromatic hydroxyaldehydes" Seite 579, Abstract Nr. 179705w**

**Chemical Abstracts Band 75, Nr. 11, 13. September 1971 Columbus, Ohio, USA S. TSUTSUMI et al. "Benzoylformic acid" Seite 435, Abstract Nr. 76420f**

(73) Patentinhaber: **BAYER AG**
**Zentralbereich Patente, Marken und Lizenzen**
**D - 5090 Leverkusen 1, Bayerwerk (DE)**

(72) Erfinder: **Fiege, Helmut, Dr.**
**Walter-Flex-Strasse 8**
**D - 5090 Leverkusen 1 (DE)**
**Wedemeyer, Karlfried, Dr.**
**Bilharzstrasse 7,**
**D - 5000 Koeln 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 008 699 B1

# Verfahren zur Herstellung von Arylglyoxylsäuren

Die vorliegende Erfindung betrifft ein Verfahren zur Herstellung von Arylglyoxysäuren durch Oxidation von Aryläthan-1,2-diol-Verbindungen mit Sauerstoff oder Sauerstoff enthaltenden Gasen in wäßrig-alkalischen Medien bei Temperaturen bis zum Siedepunkt des Reaktionsgemisches in Gegenwart von Platinmetall-Katalysatoren. Arylglyoxysäuren sind wertvolle Zwischenprodukte in der organischen Synthese, z.B. bei der Herstellung von Pflanzenschutzmitteln.

Die Oxidation von Phenyläthan-1,2-diol zu Phenylglyoxylsäure kann formal über folgende Zwischenstufen verlaufen (s. Rodd's Chemistry of Carbon Compounds, Vol. III Part E, 2. Auflage (1974) S. 71 ff.):

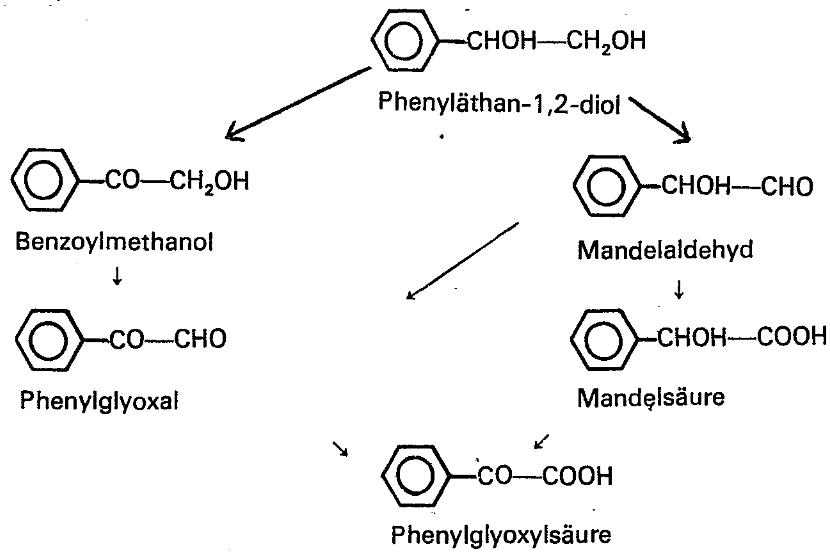

Phenyläthan-1,2-diol

Benzoylmethanol

Mandelaldehyd

Phenylglyoxal

Mandelsäure

Phenylglyoxylsäure

Jede dieser Verbindungen ist strukturell für zahlreiche Nebenreaktionen, insbesondere für oxidative Spaltungen (zu Benzaldehyd, Benzoesäure, Formaldehyd, Ameisensäure und/oder $CO_2$), prädestiniert. Die Chance, in einem Schritt durch Oxidation von Phenyläthan-1,2-diol selektiv zur Phenylglyoxylsäure zu kommen, ist somit äußerst gering. Dies bestätigen die literaturbekannten Ergebnisse: Während Mandelsäure — wenn auch nur in schlechter Ausbeute — zu Phenylglyoxylsäure oxidiert werden kann (vgl. Organic Synthesis, Coll. Vol. I, 2. Aufl. 1956, S.241 bis 245), führt die Oxidation von Phenyläthan-1,2-diol mit Kaliumpermanganat nahezu vollständig zu Benzoesäure (vgl. J. Am. Chem. Soc. *35* (1913) S. 54—68). Im wesentlichen oxidative spaltung tritt auch bei Oxidation von Phenyläthan-1,2-diol mit Chromsäure, Kalium-hexacyanoferrat (III), Silberoxid, Brom (s. vorstehendes Zitat) oder bei der Oxidation mit Nickelperoxid (vgl. Chem. Pharm. Bull. (Tokyo) *12* (1964) 403—7), mit Kaliumperjodat (vgl. Talanta *23* (1976), S. 237—9) oder mit Blei-tetra-acetat (s. Rodd's Chemistry of Carbon Compounds, Vol. III Part E, 2. Auflage (1974), S. 74) ein. Mit Salpetersäure bleibt die Oxidation unter milden Bedingungen auf der Stufe des Benzoylmethanols stehen; unter verschärften, technisch wenig attraktiven Bedingungen wurde qualitativ Benzoylameisensäure neben Benzoesäure festgestellt, (Liebigs Annalen der Chemie *216* (1883), S. 313; Ber. Dtsch. Chem. Ges. *10* (1877) S. 1488). Durch elektrochemische Oxidation von Phenyläthan-1,2-diol konnte selbst unter gunstigsten Bedingungen (platinierte Platinanoden) Phenylglyoxylsäure nur in untergeordneten Mengen (neben Mandelsäure, Benzoesäure, Ameisensäure und $CO_2$) erhalten werden (vgl. Ann. Acad. Sci. fenn. [A] *39* Nr. 11 (1934), S. 63).

Es ist mithin kein Verfahren bekannt, das eine selektive Oxidation von Phenyläthylen-1,2-glycol zu Phenylglyoxylsäure unter technisch interessanten Bedingungen gestattet.

Es wurde nun gefunden, daß man Arylglyoxylsäuren in sehr hoher Ausbeute und ausgezeichneter Reinheit erhält, wenn man Aryläthan-1,2-diole der Formel

$$\begin{array}{lll} Ar—CH—CH_2 & & \\ \quad\;\; | \quad\;\; | & & \\ \quad\; OH \;\; OH & & (I) \end{array}$$

in der

Ar für einen gegebenenfalls substituierten Arylrest steht, mit Sauerstoff oder molekularen Sauerstoff enthaltenden Gasen in wäßrig-alkalischem Medium in Gegenwart von Platinmetall-Katalysatoren bei gleichzeitiger Anwesenheit von Blei und/oder Wismut und/oder deren Verbindungen als Aktivatoren bei Temperaturen bis zum Siedepunkt des Reaktionsgemisches oxidiert.

Die Aryläthan-1,2-diole (I) können in der R—, der S— oder der (±)-Form vorliegen. Auch Gemische verschiedener Aryläthan-1,2-diole können zur Oxidation eingesetzt werden.

Es ist im Hinblick auf den Stand der Technik als ausgesprochen überraschend zu bezeichnen, daß die sonst übliche oxidative Spaltung unter den Bedingungen des erfindungsgemäßen Verfahrens weitgehend unterbleibt und es in technisch einfacher Weise gelingt, Aryläthan-1,2-diole, wie z.B. Phenyläthan-1,2-diol (Phenyläthylen-1,2-glycol), mit sehr hoher Ausbeute und Reinheit, also hochselektiv, in Arylglyoxylsäuren wie z.B. Phenylglyoxylsäure, zu überführen.

Darüber hinaus weist das erfindungsgemäße Verfahren eine Reihe weiterer Vorteile, auf: So wird als Oxidationsmittel Sauerstoff verwendet, der allgemein zur Verfügung steht, billig ist und nicht zu umweltbelastenden Oxidationsmittel — Folgeprodukten führt. Infolge der hohen Selektivität entstehen weniger Nebenprodukte, die abgetrennt und beseitigt werden müssen; dies bedeutet zugleich, daß die Vergeudung wertvoller Rohstoffe vermieden wird. Technische Vorteile leigen ferner darin, daß die Reaktionsbedingungen eine gute Wärmeabfuhr gestatten, der pH-Wert des Reaktionsmediums die Durchführung der Reaktion auch in Stahlapparaturen ermöglicht und die Oxidation über die Sauerstoffaufnahme gut gesteuert werden kann.

Verwendet man Phenyläthylen-1,2-glycol als Ausgangs-Verbindung, so kann der Reaktionsablauf summarisch durch das folgende Formelschema wiedergegeben werden:

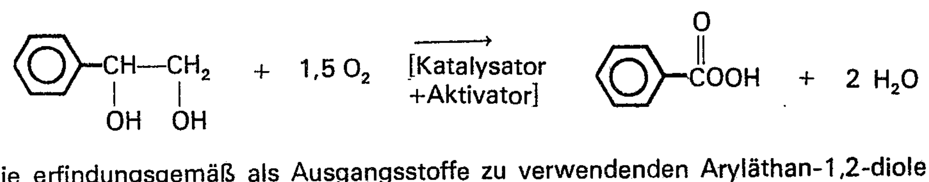


Die erfindungsgemäß als Ausgangsstoffe zu verwendenden Aryläthan-1,2-diole sind durch die Formel (I) allgemein definiert. In dieser Formel steht Ar vorzugsweise für Phenyl. Der Rest Ar steht ferner für substituiertes Aryl, wobei bevorzugt folgende Substituenten in Frage kommen: Alkyl, vorzugsweise mit 1 bis 6 C-Atomen, Cycloalkyl, vorzugsweise mit 3 bis 6 C-Atomen; Aryl, vorzugsweise Phenyl; Aralkyl, vorzugsweise Benzyl; Alkoxy, vorzugsweise mit 1 bis 6 C-Atomen; Cycloalkoxy, vorzugsweise mit 3 bis 6 C-Atomen; Aryloxy, vorzugsweise Phenoxy; Hydroxy; Carboxy und/oder Halogen (insbesondere Fluor, Chlor und/oder Brom) sowie die Methylendioxy-Gruppe. Wenn der Arylrest Phenyl bedeutet, stehen die Substituenten vorzugsweise in der 3-, 4- und/oder 5-Stellung.

Die als Ausgangsverbindungen benötigten Aryläthan -1,2-diole der Formel (I) sind bekannt oder können nach bekannten Verfahren hergestellt werden. So kann das Phenyläthylen-1,2-glycol beispielsweise durch Hydroxylierung von Styrol mit Wasserstoffperoxid (vgl. Helv. chim. Acta *50* (1967), S. 319—321) oder durch Hydrolyse von Styroloxid hergestellt werden (Weitere Verfahren vgl. Rodd's Chemistry of Carbon Compoundes, Vol. III Part E, 2. Auflage (1974), S. 72 ff).

Unter "wäßrig-alkalischem Medium" ist zu verstehen, daß das Reaktionsgemisch alkalisch reagiert, also einen pH-Wert >7 aufweist. Vorteilhafterweise wird das Alkali so bemessen, daß auf 1 Mol zu oxidierendes Aryläthan-1,2-diol 0,3 bis 5, vorzugsweise 0,5 bis 3 Äquivalente Alkali kommen. Insbesondere bevorzugt werden etwa 0,9 bis 2 Äquivalente Alkali je Mol zu oxidierendes Aryläthan1,2-diol angewendet.

Das Alkali kann zur Lösung bzw. Suspension des Aryläthan-1,2-diols in Wasser gegeben werden, oder das Aryläthan1,2-diol kann in der Alkali-Lösung gelöst bzw. suspendiert werden.

Als Alkali werden bevorzugt Hydroxide oder Carbonate des Natriums oder Kaliums eingesetzt.

Die Konzentration der organischen Verbindungen in der wäßrig-alkalischen Reaktionslösung wird im allgemeinen so gewählt, daß sowohl das Aryläthan-1,2-diol als auch die gebildete Arylglyoxysäure unter den Reaktionsbedingungen gelöst vorliegen. Gegebenenfalls ist das Aryläthan -1,2-diol dem Oxidationsgemisch-evtl. zusammen mit einem Teil des Alkalis — portionsweise zuzuführen. Bewährt haben sich Endkonzentrationen an organischen Verbindungen im Reaktionsgemisch von 5 bis 30 Gew.%.

Unter den erfindungsgemäßen Bedingungen kann eine Oxidationswirkung bei allen Temperaturen beobachtet werden, bei denen eine flüssige Phase vorliegt. Dementsprechend reicht die mögliche Reaktionstemperatur vom Erstarrungspunkt bis zum Siedepunkt des Reaktionsgemisches. Bevorzugt wird im Temperaturbereich von 10° bis 100°C gearbeitet.

Unter "Platinmetallen", die in dem erfindungsgemäßen Verfahren als Katalysatoren eingesetzt werden, sind die chemisch nahe verwandten in der Natur meist gemeinsam auftretenden Metalle Platin, Palladium, Rhodium, Iridium, Ruthenium und Osmium zu verstehen. Bevorzugt werden die Platinmetalle Platin und Palladium eingesetzt, insbesondere Platin.

Die als Katalysatoren verwendeten Platinmetalle können den Reaktionskomponenten in verschiedenster Form zugegeben werden, beispielsweise in elementarer, d.h. metallischer, Form, z.B. als sogenanntes Mohr, in Kombination mit anderen Platinmetallen oder in Form von Verbindungen, z.B. als Oxide oder auch in Form anderer Verbindungen.

Die Platinmetalle können auch auf Träger aufgebracht sein. Als Träger geeignet sind beispielsweise Aktivkohle, Graphit, Kieselgur, Kieselgel, Spinelle, Aluminiumoxid, Asbest, Calciumcarbonat, Magnesiumcarbonat, Bariumsulfat oder auch organische Trägermaterialen. Besonders bewährt haben sich Aktivkohlen, beispielsweise aus Holz hergestellte billige pulverförmige Aktivkohlen, die vielfach für Entfärbungszwecke verwendet werden.

Der Platinmetall-Gehalt dieser Trägerkataysatoren kann in weiten Grenzen schwanken. Besonders bewährt haben sich Trägerkatalysatoren mit einem Platinmetall-Gehalt unter 10 Gew.%, insbesondere solche mit Gehalten von 0,1 bis 5 Gew.% Platinmetall.

Die Mengen, in denen die Platinmetall-Katalysatoren verwendet werden, können in weiten Grenzen schwanken. Die Mengen hängen von der gewünschten Oxidationsgeschwindigkeit, der Katalysatorform, der Aktivator-Art und -Menge usw. ab und lassen sich im speziellen Fall durch Vorversuche leicht ermitteln.

Im allgemeinen liegt die je Mol Aryläthan-1,2-diol erforderliche Platinmetall-Menge unter 1000 mg. in den meisten Fällen werden mit 20 bis 400 mg Platinmenge je Mol Aryläthan-1,2-diol ausreichend hohe Reaktionsgeschwindigkeiten erreicht.

Da bei Verwendung der erfindungsgemäßen Aktivatoren eine Teerbildung fast vollständig vermieden wird, können die Katalysatoren wiederholt eingesetzt werden. Durch diese Wiederverwendung kann der Verbrauch an Platinmetall-Katalysator je Mol Aryläthan-1,2-diol auf 5 mg und darunter gesenkt werden, bevor eine Wiederaufarbeitung des Platinmetall-Katalysators erforderlich wird.

Als Aktivatoren haben sich vor allem Blei und/oder Wismut bewährt. Die Mengen, in denen die erfindungsgemäß zu verwendenden Aktivatoren eingesetzt werden, können in weiteren Grenzen schwanken. Die Aktivatorwirkung macht sich bereits bei Zusätzen von $1 \times 10^{-5}$ Mol Metall bzw. Metallverbindung je Mol Aryläthan-1,2-diol deutlich bemerkbar. Es können auch 0,1 Mol oder mehr Aktivator je Mol Aryläthan-1,2-diol eingesetzt werden, jedoch bieten diese hohen Zusätze im allgemeinen keinen Vorteil. Im allgemeinen haben sich Zusätze von $5 \times 10^{-5}$ bis $1 \times 10^{-1}$ Mol, vorzugsweise $1 \times 10^{-4}$ bis $1 \times 10^{-2}$ Mol Metall je Mol zu oxidierendes Aryläthan-1,2-diol bewährt.

Die erfindungsgemäß als Aktivatoren zu verwendenden Metalle können als solche, d.h. in elementarer Form und/oder in Form ihrer Verbindungen, z.B. als Oxide oder Salze von Wasserstoffsäuren, wie Chloride, Bromide, Jodide, Sulfide, Selenide, Telluride, oder als Salze von anorganischen Sauerstoffsäuren, wie Nitrate, Nitrite, Phospite, Phosphate, Carbonate, Perchlorate, Antimonate, Arseniate, Selenite, Sulfate, Seleniate, Borate, oder als Salze von Sauerstoffsäuren, die von Übergangsmetallen abstammen, wie z.B. Vanadate, Niobate, Tantalate, Chromate, Molybdate, Wolframate, Permanganate, oder als Salze organischer aliphatischer oder aromatischer Säuren wie z.B. Formiate, Acetate, Propionate, Benzoate, Salicylate, Lactate, Mandelate, Glyoxylate, Arylglyoxylate, Citrate oder als Phenolate usw. eingesetzt werden. Die Aktivatoren können im Reaktionsgemisch jeweils löslich, teilweise löslich oder unlöslich sein.

Auch Kombinationen dieser Aktivatoren untereinander und/oder mit anderen, nicht als Aktivator beanspruchten Elementen oder Verbindungen können verwendet werden. Die erfindungsgemäßen Aktivatoren können in unterschiedlichen und auch gemischten Wertigkeitsstuffen vorliegen; auch können Änderungen in der Wertigkeit während der Reaktion eintreten. Sofern die Aktivatoren nicht bereits als Oxide und/oder Hydroxide zugegeben werden, ist es möglich, daß sie sich im alkalischen Medium ganz oder teilsweise in diese umwandeln. Nach der Reaktion kann der Platinmetall-Katalysator mit dem schwerlölichen Aktivator abfiltriert und in weiteren Oxidationen wiederverwendet werden. Verluste an Platinmetall-Katalysatoren und/oder Aktivator sind gegebenenfalls zu ersetzen.

Der Aktivator kann als Feststoff, vorzugsweise in fein verteilter Form, oder in gelöster Form den Reaktionskomponenten zugesetzt werden. Man kann den Aktivator auch schon bei der Herstellung des Platinmetall-Katalysators zugeben oder den Platinmetall-Katalysator mit dem Aktivator imprägnieren. Der Aktivator kann auch als Trägermaterial für das Platinmetall dienen.

Besonders bewährt hat sich die Kombination von Platin mit Blei und/oder Wismut.

Das erfindungsgemäße Verfahren wird üblicherweise so durchgeführt, daß man Sauerstoff oder molekularen Sauerstoff enthaltende Gase wie Luft mit der das alkalische Mittel, den Platinmetall-Katalysator und den erfindungsgemäßen Aktivator enthaltenden Lösung des Aryläthan-1,2-diols in guten Kontakt bringt. Gewöhnlich arbeitet man bei Atmosphärendruck (1 bar), jedoch kann auch bei höheren oder niedrigeren Drucken oxidiert werden, beispielsweise im Bereich von 0,5 bis 10 bar. Der Verlauf der Reaktion kann über die aufgenommene Sauerstoffmenge verfolgt werden und wird abgebrochen, wenn die für die gewünschte Arylglyoxylsäure theoretisch erforderliche Sauerstoffmenge aufgenommen ist. Meistens hört die Sauerstoffaufnahme in diesem Stadium von selbst auf oder sie verlangsamt sich. Der Fortgang der Reaktion kann auch auf andere Weise, z.B. durch Bestimmung der gebildeten Arylglyoxylsäure verfolgt werden.

Zur Aufarbeitung werden Platinmetall-Katalysator nebst ungelöstem Aktivator vom Reaktionsgemisch abgetrennt, beispielsweise durch Filtrieren. Aus der alkalischen Reaktionslösung wird die Arylglyoxysäure durch Ansäueren auf einen pH-Wert unterhalb von 6 freigesetzt und nach bekannten Verfahren wie Dekantieren, Abfiltrieren und/oder Extrahieren abgetrennt und erforderlichenfalls z.B. durch Umkristallisieren, Destillieren oder Extrahieren weiter gereinigt.

Die Reihenfolge, in der Platinmetall-Katalysator, Aktivator, Alkali und Aryläthan-1,2-diol zusammengegeben werden, ist beliebig. So können Platinmetall-Katalysator und Aktivator der wäßrigalkalischen Aryläthan-1,2-diol-Lösung zugesetzt werden; man kann auch Platinmetall-Katalysator und Aktivator vorlegen und wäßrigalkalische Aryläthan-1,2-diol-Lösung zusetzen; schließlich ist es auch möglich, Platinmetall-Katalysator, einen Teil des wäßrigen Alkalis und den Aktivator vorzulgegen und das Aryläthan-1,2-diol zusammen mit dem restlichen Alkali zuzusetzen. Ferner ist es möglich, den Akti-

vator der Mischung der Reaktionskomponenten zuzugeben.

Die nach dem erfindungsgemäßen Verfahren herstellbaren Arylglyoxylsäuren sind wichtige organische Zwischenprodukte und von großer Bedeutung z.B. für die Herstellung von Pflanzenschutzmitteln, ferner von lichthärtenden Lacken sowie von Arzneimitteln.

So läßt sich beispielsweise ausgehend von Phenylglyoxylsäure der herbidzide Wirkstoff 3-Methyl-4-amino-6-phenyl-1,2,4-triazin-5 (4H)-on (vgl. DE—OS 2 224 161) herstellen.

Das erfindungsgemäße Verfahren wird durch die nachfolgenden Herstellungsbeispiele veranschaulicht:

## Herstellungsbeispiele

### Beispiel 1

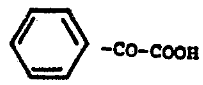

In einem mit Rührer, Thermometer und Gaszuleitung versehenen Reaktionsgefäß werden 2 g platinhaltige Aktivkohle (Platingehalt: 1 Gew.%), 1,5 ml 0,1-molare $Pb(NO_3)_2$-Lösung (entsprechend einer Blei-Menge von $1,5 \times 10^{-4}$ Mol) und eine Lösung von 13,8 g (0,1 Mol) (±)-Phenyläthylen-1,2-glycol in 100 ml 1,3 n Natronlauge eingebracht.

Nach Verdrängen der Luft aus dem Reaktionsgefäß durch Sauerstoff wird der Rührer angestellt und bei 70°C unter kräftigem Rühren reiner Sauerstoff unter Normaldruck in die Mischung eingeleitet. Nach 45 Minuten sind 0,15 Mol $O_2$ aufgenommen und die Sauerstoffaufnahme kommt nahezu zum Stillstand.

Nach Abfiltrieren des Kontaktes wird im Filtrat der Gehalt an Phenylglyoxylsäure durch Differential-Puls-Polarographie bestimmt. Als Grundelektrolyt diente 1 n LiOH. Die Bestimmung erfolgte gegen Phenylglyoxylsäure-Lösung bekannten Gehaltes, die bei einer Wiederholungsmessung als interner Standard zugegeben Wurde. Die Bestimmung ergab eine Phenylglyoxylsäure-Ausbeute von 93% d.Th.

Die Phenylglyoxysäure kann auch durch Ansäuren mit Schwefelsäure freigsetzt und — ggf. nach Abfiltrieren der in geringer Menge mitentstandenen Benzoesäure (0,6 g ≙ 5% d. Th). — z.B. mit Äther, aus der Lösung extrahiert und nach Verdampfen des Äthers in freier Form enthalten werden. Der abfiltrierte Kontakt kann wiederverwendet werden.

### Beispiel 2

Es wird wie in Beispiel 1 gearbeitet, jedoch mit dem Unterschied, daß nicht Blei-, sondern $3 \times 10^{-4}$ Mol Wismut in Form seines feingepulverten Nitrats $[Bi(NO_3)_3 . 5 H_2O]$ als Aktivator zur Reaktionsmischung gegeben werden. Nach einer Oxidationszeit von 60 Minuten ist die stöchiometrisch erforderliche Sauerstoffmenge aufgenommen und die polarographische Bestimmung ergibt eine Phenylglyoxylsäureausbeute von 90% d. Th. Der Kontakt kann nach Abfiltrieren Wiederverwendet werden.

### Beispiele 3 bis 15

Es wird wie in Beispiel 1 gearbeitet, jedoch mit dem Unterschied, daß bei 75°C und mit unterschiedlichen Aktivatoren und Aktrivatormengen gearbeitet wird:

TABELLE 1

| Beispiel Nr. | Aktivator | | O$_2$-Aufnahme | | Ausbeute |
|---|---|---|---|---|---|
| | Art | Mol Aktivator pro Mol (±)-Phenyläthylen-1,2-glycol | Zeit in Minuten | Menge in % d. Th. a) | Phenylglyoxylsäure % d. Th. |
| 3 c) | ohne Aktivator | | ca. 240 | 75 b) | 10 |
| 4 | $Pb(NO_3)_2$ | $1 \times 10^{-5}$ | 90 | 87 b) | 37 |
| 5 | ,, | $5 \times 10^{-5}$ | 90 | 90 b) | 48 |
| 6 | ,, | $2{,}5 \times 10^{-4}$ | 60 | 98 b) | 76 |
| 7 | ,, | $5 \times 10^{-4}$ | 60 | 100 | 90 |
| 8 | ,, | $3 \times 10^{-3}$ | 40 | 100 | 93 |
| 9 | ,, | $6 \times 10^{-3}$ | 45 | 100 | 92 |
| 10 | $Bi(NO_3)_3 . 5\ H_2O$ | $2 \times 10^{-5}$ | 120 | 86 b) | 48 |
| 11 | ,, | $2 \times 10^{-4}$ | 45 | 100 | 87 |
| 12 | ,, | $1 \times 10^{-3}$ | 50 | 100 | 87 |
| 13 | Pb-Pulver | $2 \times 10^{-3}$ | 75 | 100 | 90 |
| 14 | $Pb_3O_4$ (Mennige) | $4 \times 10^{-3}$ | 125 | 100 | 89 |
| 15 | $(CH_3COO)_2Pb$ | $5 \times 10^{-4}$ | 60 | 100 | 90 |

a) 100% d. Th.=1,5 Mol $O_2$/Mol (±)-Phenyläthylen-1,2-glycol
b) Reaktion kommt nach Aufnahme dieser $O_2$-Menge praktisch zum Stillstand
c) Vergleichsbeispiel

Wie das (Vergleichs-) Beispiel 3 zeigt, verläuft die Oxidation ohne Aktivatorzugabe erheblich langsamer. Die Sauerstoffaufnahme kommt vorzeitig zum Stillstand, und die Phenylglyoxylsäureausbeute beträgt nur 10% d. Th.

Beispiel 16

Es wird wie in Beispiel 1 gearbeitet. Eingesetzt wird eine Lösung von 13,8 g (0,1 Mol) (±)Phenyläthylen-1,2-glycol in 100 ml 2 n Natronlauge. Zur Lösung werden 1,5 g Aktivkohle (Medicinalkohle) mit 5% Palladium-Gehalt sowie 2 x $10^{-4}$ Mol Bi $(No_3)_3 . 5\ H_2O$ gegeben. Dann wird bei 70°C und 1 bar $O_2$-Druck oxidiert. Nach 140 Minuten sind 0,15 Mol $O_2$ aufgenommen und die Phenylglyoxylsäure-Ausbeute beträgt 45% der Theorie.

Ohne Zusatz von Wismut beträgt die Ausbeute unter sonst gleichen Bedingungen nach Aufnahme von 0,15 Mol $O_2$ nur 10% der Theorie.

Beispiel 17

Es wird wie in Beispiel 16 gearbeitet, jedoch mit dem Unterschied, daß an Stelle von Wismut 1,5 x $10^{-3}$ Mol Blei-(II)-nitrat zur Lösung gegeben werden. Die Reaktionszeit beträgt in diesem Fall 110 Minuten und die Phenylglyoxylsäure-Ausbeute 40% der Theorie.

Ohne Zusatz von Blei beträgt die Ausbeute unter sonst gleichen Bedingungen nur 10% der Theorie.

## Patentansprüche

1. Verfahren zur Herstellung von Arylglyoxylsäuren durch Oxidation von Aryläthan-1,2-diole der Formel

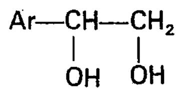

(I)

in der

Ar für einen gegebenenfalls substituierten Arylrest steht, dadurch gekennzeichnet, daß man die Oxidation mit Sauerstoff oder molekularen Sauerstoff enthaltenden Gasen in wäßrig-alkalischen Medium in Gegenwart von Platinmetall-Katalysatoren bei gleichzeitiger Anwesenheit vom Blei und/oder Wismut und/oder deren Verbindungen als Aktivatoren bei Temperaturen bis zum Siedepunkt des Reaktionsgemisches durchführt.

2. Verfahren gemäß Anspruch 1, dadurch gekennzeichnet, daß man Blei und/oder Wismut in Mengen von 5 x $10^{-5}$ bis 1 x $10^{-1}$ Mol je Mol Aryläthan-1,2-diol einsetzt.

3. Verfahren gemäß Anspruch 1, dadurch gekennzeichnet, daß man als Platinmetall-Katalysator Platin verwendet.

4. Verfahren gemäß Anspruch 1, dadurch gekennzeichnet, daß man Blei und/oder Wismut in Kombination mit Platinmetall-Katalysatoren verwendet.

5. Verfahren gemäß Anspruch 1, dadurch gekennzeichnet, daß man als Träger für das Platinmetall Aktivkohle verwendet.

6. Verfahren gemäß Anspruch 1, dadurch gekennzeichnet, daß der Platinmetallgehalt der Trägerkatalysatoren unter 10 Gew.-% liegt.

7. Verfahren gemäß Anspruch 1, dadurch gekennzeichnet, daß als Alkali Natrium- oder Kaliumhydroxid in Mengen von 0,3 bis 5 Äquivalenten, vorzugsweise von 0,5 bis 3 Äquivalenten, insbesondere von 0,9 bis 2 Äquivalenten pro Mol Aryläthan-1,2-diol eingesetzt werden.

8. Verfahren gemäß Anspruch 1, dadurch gekennzeichnet, daß man bei Temperaturen von 10°C bis 100°C arbeitet.

9. Verfahren gemäß Anspruch 1, dadurch gekennzeichnet, daß man mit Sauerstoff oder Sauerstoff enthaltenden Gasen bei einem Druck von 0,5 — 10 bar arbeitet.

10. Verfahren nach Anspruch 1, dadurch gekennzeichnet, daß man als Aryläthan-1,2-diol Phenyläthylen-1,2-glycol einsetzt.

## Revendications

1. Procédé de fabrication d'acides arylglyoxyliques par oxydation d'aryléthane-1,2-diols de formule:

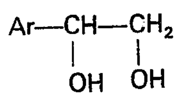

(I)

dans laquelle

Ar représente un radical aryle éventuellement substitué caractérisé en ce qu'on exécute l'oxydation avec de l'oxygène ou des gaz contenant de l'oxygène moléculaire en milieu aquo-alcalin en présence de catalyseurs de métaux du platine avec présence simultanée de plomb et/ou de bismuth et/ou de leurs composés comme activateurs à des températures allant jusqu'au point d'ébullition du mélange de réaction.

2. Procédé selon la revendication 1, caractérisé en ce qu'on utilise le plomb et/ou le bismuth en des quantitiés de $5 \times 10^{-5}$ à $1 \times 10^{1}$ mole par mole d'aryléthane-1,2-diol.

3. Procédé selon la revendication 1, caractérisé en ce qu'en tant que catalyseur de métaux du platine on utilise du platine.

4. Procédé selon la revendication 1, caractérisé en ce qu'on utilise le plomb et/ou le bismuth en combinaison avec les catalyseurs du platine.

5. Procédé selon la revendication 1, caractérisé en ce qu'on utilise du charbon actif comme support pour les métaux du platine.

6. Procédé selon la revendication 1, caractérisé en ce que la teneur en métaux du platine des catalyseurs supportés est inférieure à 10% en poids.

7. Procédé selon la revendication 1, caractérisé en ce qu'on utilise en tant qu'alcali de l'hydroxyde de sodium ou de potassium en des quantités de 0,3 à 5 équivalents, de préférence de 0,5 à 3 équivalents, en particulier de 0,9 à 2 équivalents par mole d'aryléthane-1,2-diol.

8. Procédé selon la revendication 1, caractérisé en ce qu'on opère à des températures de 10°C à 100°C.

9. Procédé selon la revendication 1, caractérisé en ce qu'on opère avec de l'oxygène ou des gaz contenant de l'oxygène sous une pression de 0,5 à 10 bars.

10. Procédé selon la revendication 1, caractérisé en ce qu'on utilise de phényléthyléne-1,2-glycol en tant qu'aryléthane-1,2-diol.

## Claims

1. Process for the preparation of arylglyoxylic acids by oxidation of arylethane-1,2-diols of the formula

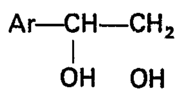

(I)

in which

Ar represents an optionally substituted aryl radical, characterised in that the oxidation is carried out with oxygen or gases containing molecular oxygen in an aqueous alkaline medium in the presence of platinum metal catalysts and in the simultaneous presence of lead and/or bismuth and/or their compounds as activators, at temperatures up to the boiling point of the reaction mixture.

2. Process according to Claim 1, characterised in that lead and/or bismuth in amounts of $5 \times 10^{-5}$ to $1 \times 10^{-1}$ mol per mol of arylethane-1,2-diol is employed.

3. Process according to Claim 1, characterised in that platinum is used as the platinum metal catalyst.

4. Process according to Claim 1, characterised in that lead and/or bismuth is used in combination with platinum catalysts.

5. Process according to Claim 1, characterised in that active charcoal is used as the support for the platinum metal.

6. Process according to Claim 1, characterised in that the platinum metal content of the supported catalysts is less than 10% by weight.

7. Process according to Claim 1, characterised in that the alkali employed is sodium hydroxide or potassium hydroxide in amounts of 0.3 to 5 equivalents, preferably of 0.5 to 3 equivalents, especially of 0.9 to 2 equivalents, per mol of arylethane-1,2-diol.

8. Process according to Claim 1, characterised in that it is carried out at temperatures of 10°C to 100°C.

9. Process according to Claim 1, characterised in that it is carried out with oxygen or oxygen-containing gases at a pressure of 0.5—10 bar.

10. Process according to Claim 1, characterised in that the arylethane-1,2-diol employed is phenylethylene-1,2-glycol.